# EUROPEAN PATENT APPLICATION

(11) **EP 0 823 238 A1**
(43) Date of publication of application: **11.02.1998**
(21) Application number: 97113517.3
(22) Date of filing: 05.08.1997
(51) Int. Cl.: A61B 5/024

(54) **Pulse wave detecting device**

(30) Priority: 06.08.1996 JP 206916/96
(71) Applicant: OMRON CORPORATION, Kyoto-shi, Kyoto 616 (JP)
(72) Inventor: Itonaga, Kazunobu, c/o Omron Corporation, Kyoto-shi, Kyoto (JP); Shirasaki, Osamu, c/o Omron Corporation, Kyoto-shi, Kyoto (JP); Yanagi, Shinsaku, c/o Omron Corporation, Kyoto-shi, Kyoto (JP)
(74) Representative: WILHELMS, KILIAN & PARTNER Patentanwälte

(57) **Abstract**

A pulse wave detecting device includes a main body (10), a pulse wave detection section (11) disposed on the main body for detecting a pulse wave from a finger (F), a movable member (12) having a pulse wave sensor (13) movably disposed in the pulse wave detection section (11) to be put by an inner side of the finger (F), and a spring (14) disposed in the main body (10) for biasing the movable member (12) to the inner side of the finger (F). As the finger (F) is inserted into the pulse wave detection section (11) and the inner side of the finger (F) is put on the movable member (12), the movable member (12) is biased to the finger by the spring (14).

## Description

### BACKGROUND OF THE INVENTION

### 1. Field of the Invention

This invention relates to a pulse wave detecting device for detecting a pulse wave obtained from a finger, and more particularly to an improved device having a construction to precisely detect the pulse wave.

### 2. Discussion of the Related Art

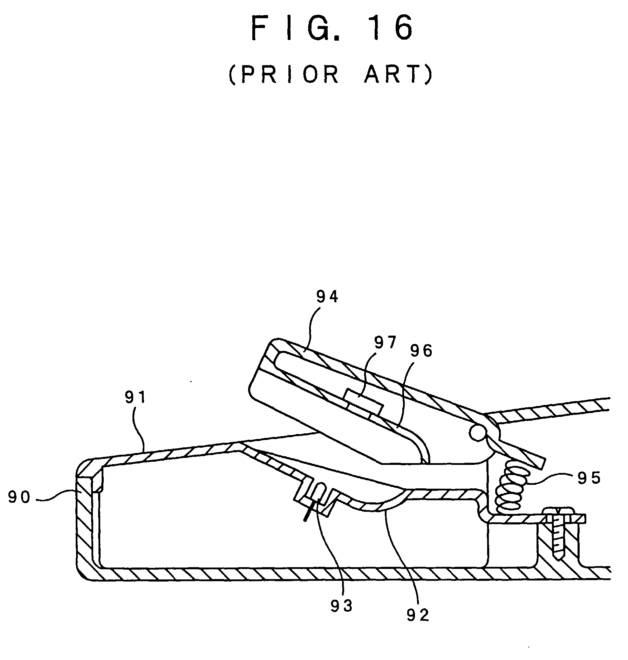

Referring to Fig. 16, there is shown a conventional pulse wave measuring device disclosed in the Japanese Laid-open Patent Publication Hei 7-213498, in which a sensor table 91 is fixed to a main body 90 and provided with a groove 92 which has a concave configuration fitting to an inner side of a finger and is installed by a light emitting element 93. A sensor lid 94 is mounted to the sensor table 91 and biased to the same by a coil spring 95. The lid 94 is further provided with a groove 96 which has a concave configuration 96 corresponding to a back side of the finger, and installed by a light receiving sensor 97.

As a finger is inserted into the device directing the inner side thereof to the groove 92 and the back side thereof to the groove 96, the inner side is biased to the groove 92 by the sensor lid 94, wherein light is stricken to the tip of the finger by the light emitting element 93 and the light passing through the tip of the finger is received by the light receiving element 97 to measure a pulse wave based on the quantity of the received light.

In the conventional device, the light emitting element 93 serving as a pulse wave sensor is mounted on the sensor table 91 and the tip of the finger is biased toward the light emitting element 93 by the sensor lid 94, the finger pressure is subject to the force by the finger put on the light emitting element 93. The pressure by the tip of the finger against the light emitting element 93 is not constant, and the magnitude of the pressure varies to deform the pulse wave, whereby any stable pulse wave cannot be precisely obtained. The size of the finger and the movement of its human body change the pressure affecting the measurement of pulse waves.

### SUMMARY OF THE INVENTION

It is, therefore, a primary object of this invention to provide an improved pulse wave detecting device capable of providing a precise and stable pulse wave measurement free from any affection by a finger pressure, the size of a finger and the movement of a human body.

According to a first aspect of this invention, there is provided a pulse wave detecting device including a main body, a pulse wave detection section disposed on the main body for detecting a pulse wave from a finger, a movable member movably disposed in the pulse wave detection section to be put by an inner side of the finger, a pulse wave detecting means disposed on the movable member, and biasing means disposed in the main body for biasing the movable member to the inner side of the finger.

According to a second aspect of this invention, there is provided a pulse wave detecting device which includes a main body having a grip portion to be gripped and a detection portion movably mounted on the grip portion to be put by an inner side of a finger, the detection portion being provided with pulse wave detecting means and the main body being provided with biasing means for biasing the pulse wave detecting means to the inner side of the finger.

According to the first aspect of this invention, the movable member is biased to the inner side of the tip of the finger by the biasing means when the inner side of the finger is put on the movable member of the pulse wave detection section. According to the second aspect of this invention, the detection portion is biased to the inner side of the finger by the biasing means when the inner side of the finger is put on the detection portion. In either aspects, the movable member or the detection portion provided with the pulse wave detecting means is biased to the tip of the finger, and the pressure applied to the finger is free from the pushing force by the finger, whereby the finger is biased with a predetermined force by the movable member or the detection portion and the pulse wave form is not changed by the variation of pushing force by the finger. Accordingly, the pushing force by finger, the size of finger, the movement of the human body do not affect the pulse wave measurement, so that the result of analyzing thus obtained pulse wave forms is not affected thereby and precise and stable pulse measurement is enabled.

### BRIEF DESCRIPTION OF THE DRAWINGS

Other objectives and advantages of this invention will be more readily apparent from the following detailed description provided in conjunction with the following figures, of which:
Fig. 1 shows at (a) a schematic view of a pulse wave detecting device as a first embodiment of this invention, and at (b) a partial view showing an example of a measurement;
Fig. 2 shows a modification of the device of Fig. 1;
Fig. 3 shows another modification of the device of Fig. 1;
Fig. 4 shows a side view of a pulse wave detecting device as a second embodiment of this invention;
Fig. 5 shows a front view of a pulse wave detecting device as a third embodiment of this invention;
Fig. 6 shows a modification of the device of Fig. 5;
Fig. 7 is another modification of the device of Fig. 5;
Fig. 8 shows a partial view of a pulse wave detecting device as a fourth embodiment of this invention;
Fig. 9 is a modification of the device of Fig. 8;
Fig. 10 shows a pulse wave detecting device as a fifth embodiment of this invention;
Fig. 11 is a modification of the device of Fig. 10;
Fig. 12 shows a pulse wave detecting device as a sixth embodiment of this invention;
Fig. 13 is a modification of the device of Fig. 12;
Fig. 14 shows a schematic block diagram of a circuit construction which may be employed in the foregoing embodiments;
Fig. 15 shows an example of a measured pulse wave form displayed on a display of the circuit of Fig. 14; and
Fig. 16 a schematic sectional view of a conventional pulse wave detecting device.

### DETAILED DESCRIPTION OF THE INVENTION

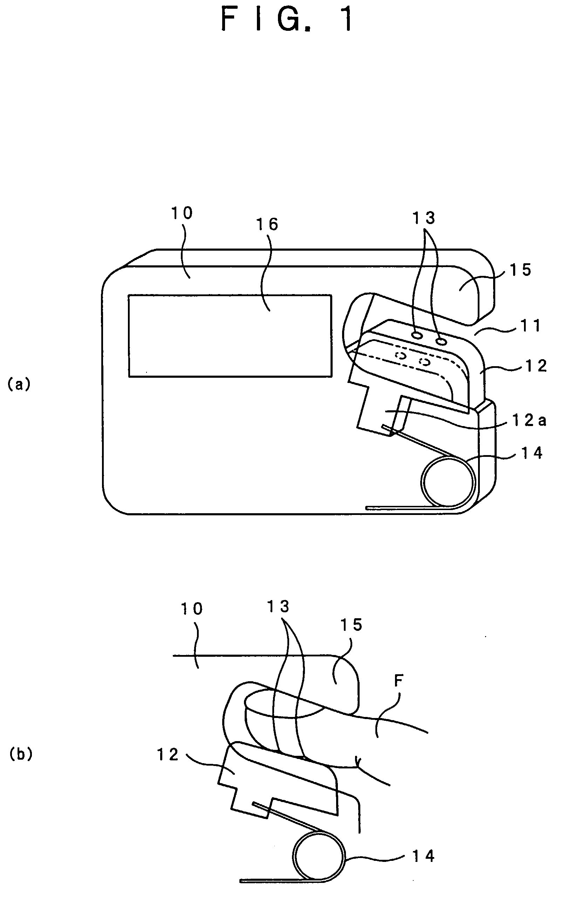

Referring, now, to Fig. 1 at (a), there is shown a schematic view of a pulse wave detecting device as a first embodiment of this invention, in which a main body 10 at one side thereof is provided with a pulse wave detection section 11 to detect a pulse wave from a finger. The detection section 11 is movably mounted by a movable member 12 which is pushed by an inner side of the finger (the tip thereof) and provided with a pulse wave sensor 13 (a pulse wave detecting means) of a photoelectric type exposed to the inner side of the finger. The main body 10 includes a bias spring 14 (a biasing means) for biasing the movable member 12 toward the inner side of the finger.

The pulse wave sensor 13 includes a couple of a light emitting element and a light receiving element, wherein light from the light emitting element is stricken to the tip of the finger, and the light receiving element receives the light reflected by the tip of the finger to measure a pulse wave based on the quantity of the received light. The bias spring 14 at one end thereof is engaged with a projection 12a of the movable member 12 and at other end thereof is engaged with an inner wall of the main body 10, whereby the movable member 12 is always biased to be exposed to the pulse wave detection section 11. The biasing force by the spring 14 is set to a force as described later. The main body 10 includes a finger guide member 15 at the pulse wave detection section 11, and a display 16 for displaying a result of the pulse measurement.

As shown in Fig. 1 at (b), when the tip of a finger F such as a forefinger is inserted into the pulse wave detection section 11 directing an inner side of the finger F to the movable member 12, the inner side of the finger is pushed by the movable member 12 biased by the spring 14 and brought into contact with the pulse wave sensor 13 slightly projecting from the movable member 12. As the finger is positioned for measurement, light is stricken to the tip of the finger F from the light emitting element, the light receiving element receives the light reflected by the finger F, and the display 16 displays the result of the pulse wave measurement based on the quantity of the received light.

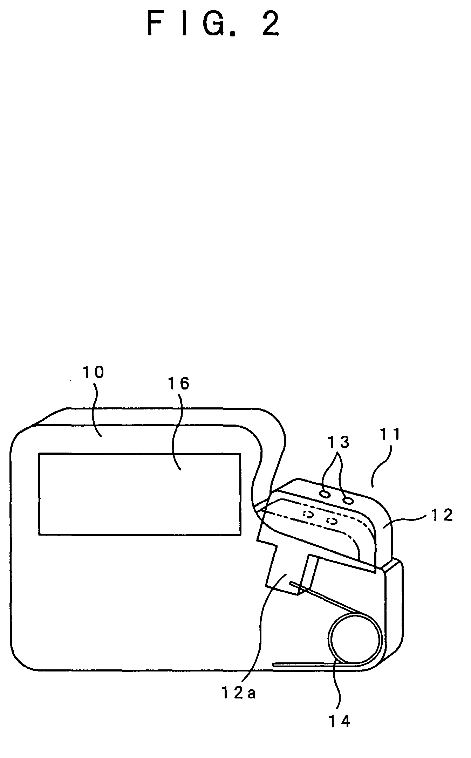

Fig. 2 shows a modification of the device of Fig. 1, in which the finger guide member 15 of Fig. 1 is removed from the pulse wave measuring section 11. Other components of Fig. 2 are the same as those of Fig. 1, and the substantial function by the device of Fig. 2 is same as that of Fig. 1.

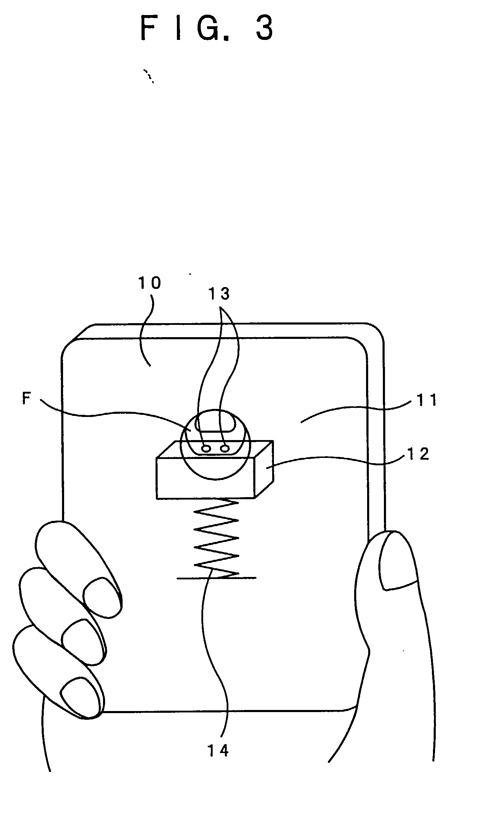

Fig. 3 shows another modification of the device of Fig. 1, in which the components corresponding to those of Fig. 1 are given by the same reference numerals. In this device of Fig. 3, main body 10 around a center thereof includes pulse wave detection section 11 to be inserted by the tip of finger F. Movable member 12 is movably installed within the body 10 at the pulse wave detection section 11, and biased toward the pulse wave detection section 11 by spring 14. Thus, the finger F is inserted into the pulse wave detection section 11 to measure a pulse wave.

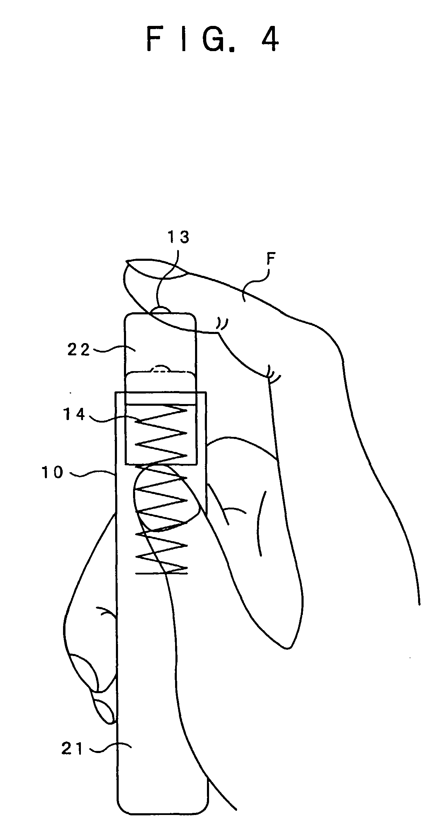

Referring to Fig. 4, there is shown a side view of a pulse wave detecting device as a second embodiment of this invention, in which a main body 10 includes a grip portion 21 gripped by fingers and a detection portion 22 put by an inner side of a finger F. The detection portion 22 is movably supported by the grip portion 21 in a vertical direction in Fig. 4, and at a top end thereof is provided with a pulse wave sensor 13 biased by a spring 14 toward a direction opposite to the grip portion 21. Thus, the pulse wave measurement is done by putting the tip of the finger F on the top end of the detection portion 22.

The pressure by the movable member 12 and the detection portion 22 by the spring 14 in the foregoing embodiments will be described hereinafter. The biasing force by the spring 14 is desirable to be lower than a diastolic blood pressure or 30 to 65 mmHg (elastic force: 40 to 90 gf). When the biasing force is designed to be lower than the diastolic blood pressure, the finger F is pushed by a force lower than the diastolic blood pressure because the pressure larger than the diastolic blood pressure deteriorates the accuracy in a blood pressure measurement. When the biasing force is designed to be 30 to 65 mmHg, the elastic force larger than 40 gf is necessary for stability against the movement of the human body and 90 gf is the maximum for pressing under a diastolic blood pressure so that the finger is biased by the pressure between 40 and 90 gf.

When the finger F is put on the movable member 12 or the detection portion 22, the force applied to the movable member 12 or the detection portion 22 by the tip of the finger happens to be too small or too large, affecting the accuracy of measurement about pulse waves. For precise measurement, the measurement may be designed to be available only when the deviation of the movable member 12 or the detection portion 22 is within a predetermined range (the force pushed by the finger is within a predetermined proper range).

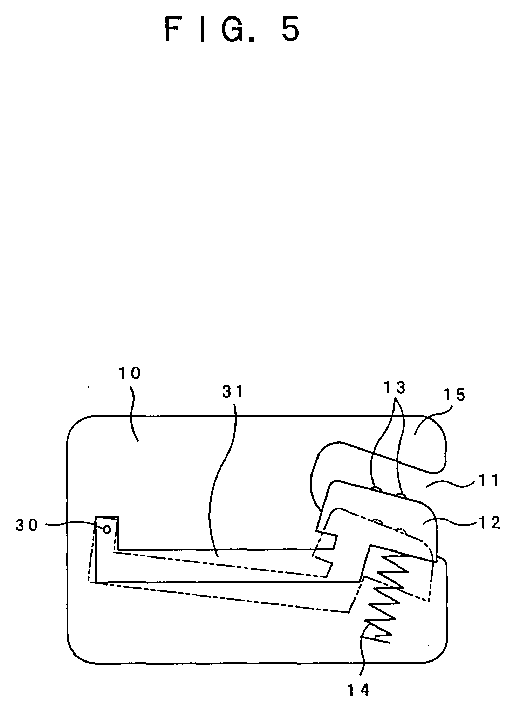

Fig. 5 shows a front view of a pulse wave detecting device as a third embodiment of this invention, in which movable member 12 is supported by a swingable lever 31 (a supporting means) pivoted about a supporting point 30. The lever 31 at one end thereof is supported by the point 30 within a main body 10 and at other end thereof provides the movable member 12 as a single unit. Thus, the movable member 12 may swing (rotate or move) centering at the supporting point 30 within a predetermined range.

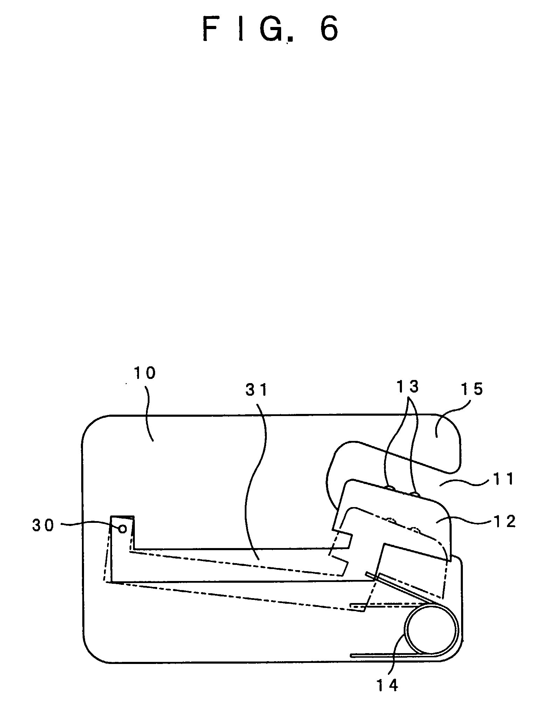

Fig. 6 shows a modification of the device of Fig. 5 in which a spring 14 of another type is employed as a biasing means and other components are the same as those of Fig. 5.

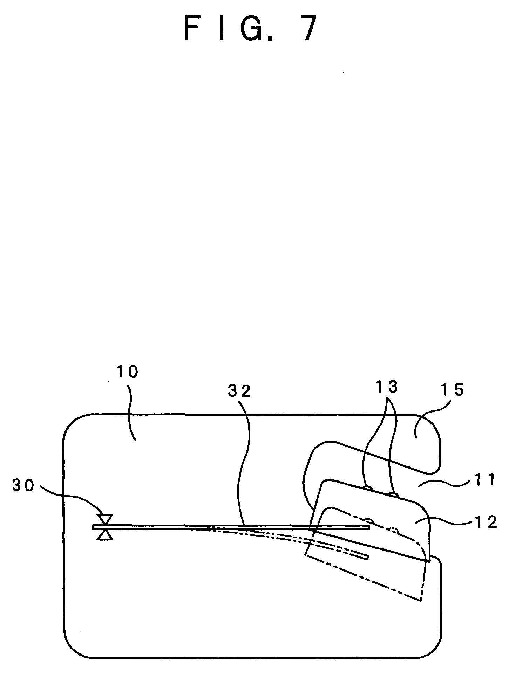

Fig. 7 is another modification of the device of Fig. 5 in which a leaf spring 32 serves as the supporting means and the biasing means. The leaf spring 32 at one end thereof is supported by a supporting point 30 and at other end thereof engaged with the movable member 12. The movable member 12 is biased by the leaf spring 32 and may move centering at the supporting point 30 within a predetermined range.

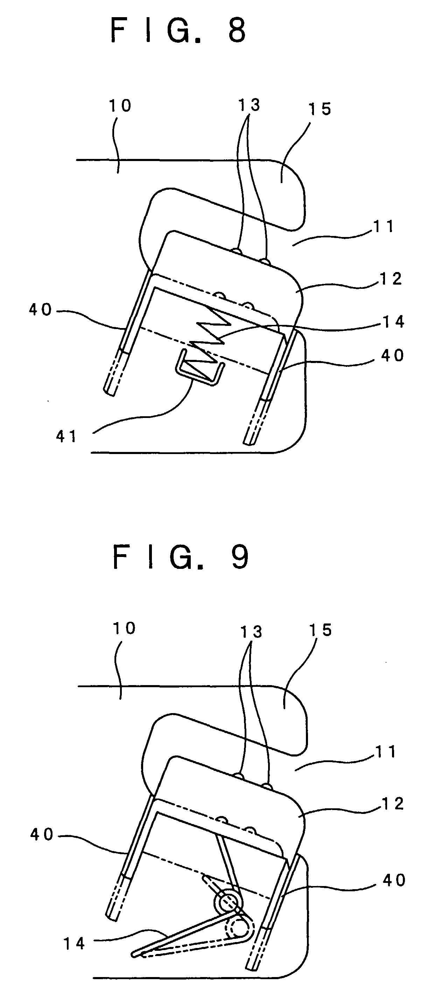

Fig. 8 shows a partial view of a pulse wave detecting device as a fourth embodiment of this invention, in which movable member 12 slidably moves along a guide 40 disposed on a main body 10. A spring 14 at one end thereof is engaged with the movable member 12 and at other end thereof is engaged with a spring engaging blade 41 fixed to the main body 10.

Fig. 9 is a modification of the device of Fig. 8 in which a spring 14 of another type is employed as the biasing means. The spring 14 at one end thereof is engaged with the movable member 12 and at another end thereof is engaged with an inner wall of the main body 10.

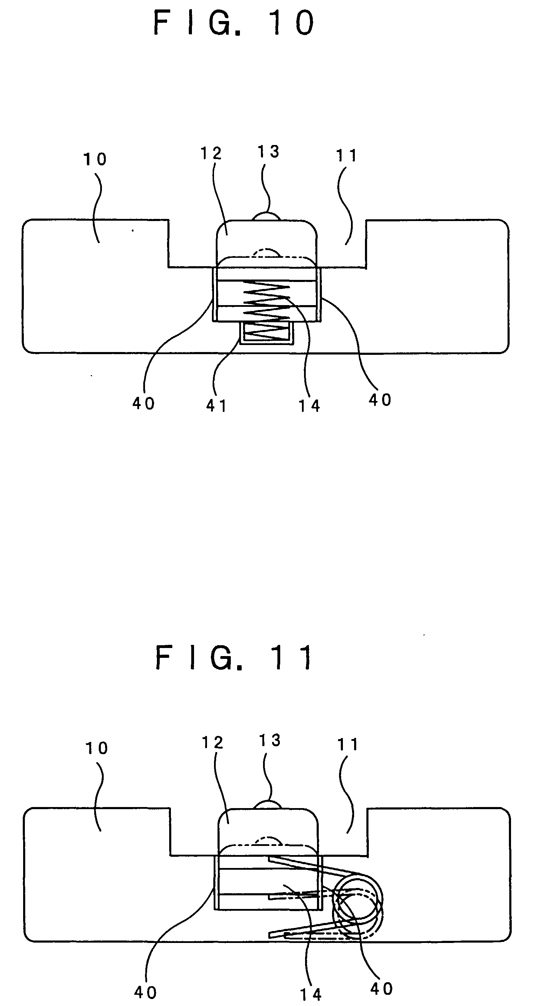

Fig. 10 shows a pulse wave detecting device as a fifth embodiment of this invention, which includes a pulse wave detection section 11 having a concave configuration in a main body 10. Movable member 12 is slidably mounted in the pulse wave detection section 11, and guided by a guide 40 disposed on the main body 10 for a slidable movement. The spring 14 at one end thereof is engaged with the movable member 12 and at other end thereof engaged with an engaging blade 41.

Fig. 11 is a modification of the device of Fig. 10 in which a spring 14 of another type is employed as the biasing means. The spring 14 at one end thereof is engaged with the movable member 12 and at another end thereof is engaged with an inner wall of the main body 10.

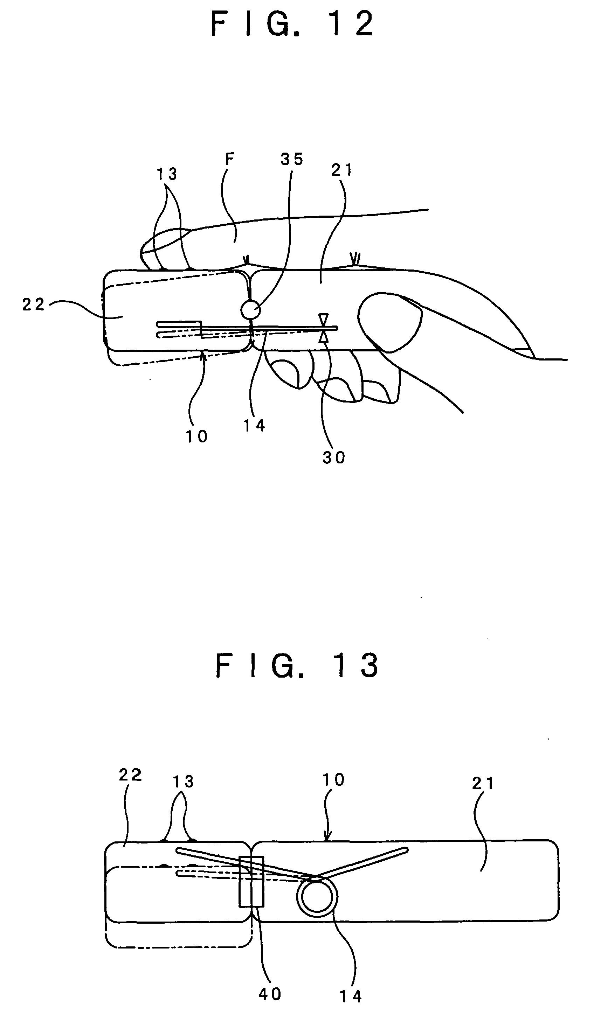

Fig. 12 shows a pulse wave detecting device as a sixth embodiment of this invention, in which a main body 10 includes a grip portion 21 and a detection portion 22 with a construction similar to the device of Fig. 4, but the detection portion 22 is connected with the grip portion 21 through a hinge 35. A leaf spring 14 serving as a biasing means at one end thereof is supported by a support point 30 and at other end thereof biases the detection portion 22 toward an inner side of a finger (F) in the detection portion. Accordingly, the detection portion 22 is swung centering at the hinge 35 within a predetermined angular range.

Fig. 13 is a modification of the device of Fig. 12, in which a main body 10 includes a grip portion 21 and a detection portion 22. The detection portion 22 is connected with the grip portion 21 by a guide 40. The guide 40 is disposed between the grip portion 21 and the detection portion 22 so that the detection portion 22 may slidably move along the guide 40. The spring at one end thereof 14 is engaged with an inner wall of the grip portion 21 and at other end thereof engaged with an inner wall of the detection unit 22, in which the detection portion 22 is biased by the spring 14.

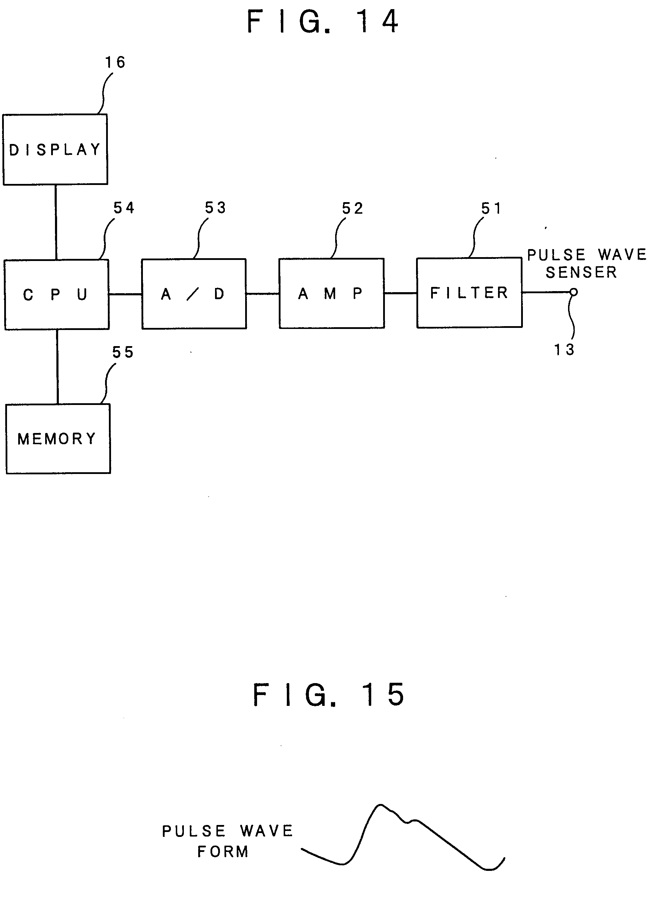

Fig. 14 shows a schematic block diagram of a circuit construction which may be employed in the foregoing embodiments. A signal from the pulse wave sensor 13 of the photoelectric type is applied to a filter 51 to remove noise of the signal, and amplified by an amplifier 52 to be applied to an A/D converter which converts the amplified signal to a digital signal. The digital signal is applied to a central processing unit 54 which controls display by a display 16. For instance, the display 16 displays a wave form of the measured pulse wave shown in Fig. 15, and a memory 55 stores the measured pulse wave for data transactions.

In the foregoing embodiments, the biasing member employs springs. If desired, other elastic members such as rubber or sponges may be employed as the biasing member. A balance type construction may be employed in which a weight is put on one plate and other plate biases a finger.

According to the foregoing embodiments, the movable member or the detection portion provided with the pulse wave detecting means is biased to the tip of the finger, and the pressure applied to the finger is free from the pushing force by the finger, whereby the finger is biased with a predetermined force by the movable member or the detection portion and the pulse wave form is not changed by the variation of the pushing force by the finger. Accordingly, the pushing force against the pulse wave sensor by finger, the size of finger, the movement of the human body do not affect the pulse wave measurement, so that the result of analyzing thus obtained pulse wave forms is not affected thereby and precise and stable pulse measurement is enabled. When the inner side of the finger is biased by a force below a diastolic blood pressure or between 30 and 65 mmHg (40 and 90 gf in elastic force), the accuracy of the pulse wave detection is improved. As the measurement is designed to be available only when the deviation of the movable member or the detection portion is within a predetermined range, too large or small pushing force by the finger is excluded from the measurement, whereby the measurement accuracy is ensured.

While the invention has been described and illustrated with respect to certain embodiments which give satisfactory results, it will be understood by those skilled in the art, after understanding the purpose of the invention, that various other changes and modifications may be made without departing from the spirit and scope of the invention, and it is therefore, intended in the appended claims to cover all such changes and modifications.

## Claims

1. A pulse wave detecting device comprising
a main body (10),
a pulse wave detection section (11) disposed on said main body (10) for detecting a pulse wave from a finger (F),
a movable member (12) movably disposed in said pulse wave detection section (11) to be put by an inner side of said finger (F),
pulse wave detecting means (13) disposed on said movable member (12), and
biasing means (14, 32) disposed in the main body (10) for biasing said movable member (12) to said inner side of the finger (F).

2. A pulse wave detecting device comprising
a main body (10) including a grip portion (21) to be gripped and a detection portion (22) movably mounted on said grip portion (21) to be put by an inner side of a finger (F),
said detection portion (22) being provided with pulse wave detecting means (13) and said main body (10) being provided with biasing means (14) for biasing said pulse wave detecting means (13) to the inner side of the finger (F).

3. A pulse wave detecting device according to claim 1 or 2, in which when said finger (F) is put on said movable member (12) or detection portion (22) for a pulse wave measurement, the inner side of the finger (F) is biased by a pressure lower than a diastolic blood pressure by said biasing means (14).

4. A pulse wave detecting device according to claim 1 or 2, in which when said finger (F) is put on said movable member (12) or detection portion (22) for a pulse wave measurement, the inner side of the finger (F) is biased by a pressure value between 30 and 65 mmHg (elastic forces 40 and 90 gf) by said biasing means (14).

5. A pulse wave detecting device according to claim 1 or 2, in which when said finger (F) is put on said movable member (12) or detection portion (22) for a pulse wave measurement, the pulse wave measurement by said device is available only when the deviation of said movable member (12) or said detection portion (22) is within a predetermined range.

6. A pulse wave detecting device according to claim 1, in which said biasing member (14) is an elastic member, and said movable member (12) is supported by a supporting member (31) which at one end is supported by a supporting point (30) and at other end supports the movable member (12) for a swingable movement centering at said supporting point (30).

7. A pulse wave detecting device according to claim 1, in which said biasing member (14) is an elastic member, and said movable member (12) is moved along a guide (40) disposed on said main body (10).

8. A pulse wave detecting device according to claim 2, in which said biasing member (14) is an elastic member, and said detection portion (22) is swingably connected with said grip portion (21) by a hinge (35).

9. A pulse wave detecting device according to claim 2, in which said biasing member (14) is an elastic member, and said detection portion (22) is connected with said grip portion (21) by a guide (40) to move the detection portion (22) along said guide (40).
